# EUROPEAN PATENT SPECIFICATION

(11) **EP 1 101 460 B1**
(45) Date of publication and mention of the grant of the patent: **20.04.2005**
(21) Application number: 00113081.4
(22) Date of filing: 26.06.2000
(51) Int. Cl.: A61F 2/36

(54) **Hip endoprosthesis**
Hüftendoprothese
Endoprothèse de la hanche

(30) Priority: 17.11.1999 IT BO990624
(43) Date of publication of application: 23.05.2001
(73) Proprietor: CITIEFFE S.r.l., 40012 Calderara di Reno (Bologna) (IT)
(72) Inventor: Mingozzi, Franco, 40012 Lippo di Calderara (Prov. Bologna) (IT)
(74) Representative: Modiano, Guido, Dr.-Ing.

(56) References cited:
- EP-A- 0 257 359
- EP-A- 0 290 735
- WO-A-96/01086
- FR-A- 2 576 793
- FR-A- 2 634 371
- FR-A- 2 767 471
- US-A- 5 653 764
- US-A- 5 725 592

## Description

The present invention relates to a hip endoprosthesis.

Current hip endoprostheses have an elongated body composed of a metaphyseal part which is intermediate between a distal part and a proximal part.

The distal part is constituted by a shaft or rod which is appropriately tapered for insertion in the medullary canal.

The end of the rod has radial slits designed to modulate the rigidity of the shaft inside the medullary canal, preventing the onset of regions of sharply different rigidity at the end of the rod.

The proximal part is constituted by a collar from which a stem protrudes obliquely; the stem is preset for the taper-fit coupling of a spherical head designed to articulately engage a complementarily shaped seat of a cotyloid element which is anchored in the pelvic bone.

The collar is fixed to the metaphyseal part by means of a taper-fit coupling which allows its orientation with respect to the longitudinal axis of the endoprosthesis.

Conventional endoprostheses suffer substantial drawbacks which compromise or limit their effectiveness.

First of all, it must be noted that conventional endoprostheses are externally provided with longitudinal ridges designed to provide a rotation-preventing engagement in the medullar canal. However, these ridges generate hyperpressure regions affecting the bone cortex, leading to reactive osteolysis and weakening of the stability of the prosthetic implant.

Furthermore, during walking the loads on the prosthesis create moments which lead to undue compressive stresses on the bone cortex produced by the collar at the region that lies between the stem and the metaphyseal part.

FR-A-2 767 471 discloses a hip endoprosthesis having a combination of features as set forth in the precharacterizing portion of the appended claim 1. The aim of the present invention is to provide a hip endoprosthesis which allows to obviate the drawbacks of those currently in use.

Within the scope of this aim, an object of the present invention is to provide a hip endoprosthesis whose structure has interchangeable modules, such as to allow a wide range of assembly combinations which can be adapted to the various femoral implantation requirements.

These and other objects are achieved with a hip endoprosthesis as defined in the appended claims.

Further characteristics of the present invention will become better apparent from the following detailed description of a preferred embodiment, illustrated only by way of non-limitative example in the accompanying drawings, wherein:
Figure 1 is a longitudinal sectional view of a hip endoprosthesis having a monolithic structure;
Figure 2 is an axial sectional view, taken along the line II-II of Figure 1;
Figure 3 is a sectional view, taken along the line III-III of Figure 1;
Figure 4 is a side view of a hip endoprosthesis of the modular type; and
Figure 5 is an exploded axial sectional view of the endoprosthesis of Figure 4.

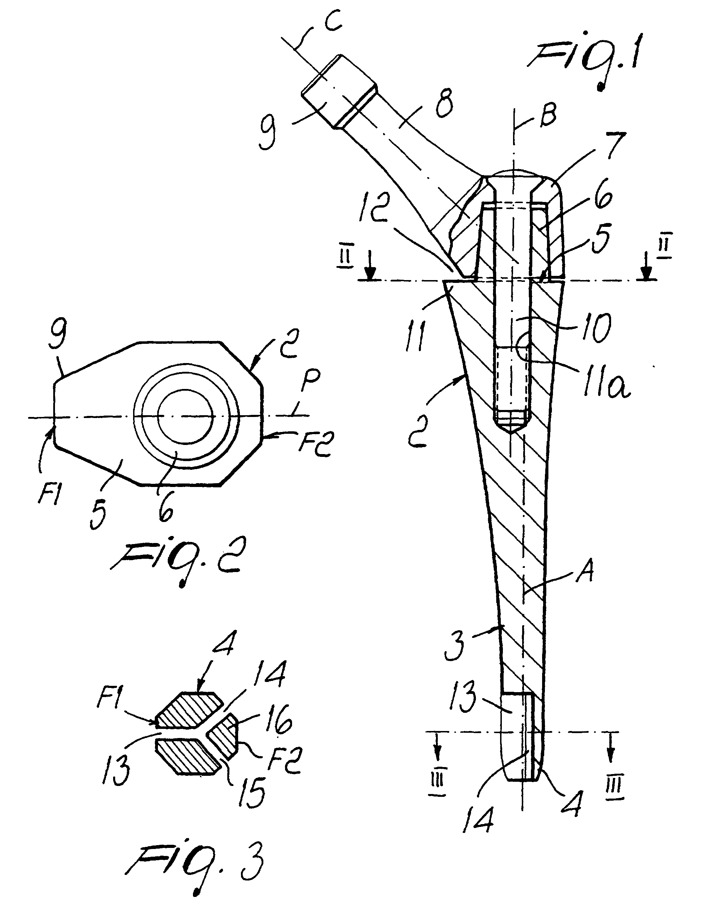

With reference to Figures 1 and 2, the reference numeral 1 designates a hip endoprosthesis according to the present invention. The endoprosthesis comprises a metaphyseal portion 2, from which a rod-shaped portion 3 protrudes; said portion is termed rod hereinafter, has an axis A and ends with an ogive-like tip 4.

The endoprosthesis 1 has an octagonal polygonal cross-section which is flattened on the central plane P so that the two faces F1, F2 are perpendicular to the main axis and to the plane P.

The metaphyseal portion 2 is blended with the rod 3 so as to taper toward the ogive-like tip 4.

The end of the portion 2 that lies opposite the tip 4 has a plane 5 from which a tubular bush 6 rises; the bush has an axis B and is externally conical. The axis B is axially offset toward the face F1 with respect to the axis A.

A collar 7 is coupled to the tubular bush 6, and a stem 8 protrudes obliquely from the collar along the axis C and forms an obtuse angle with the metaphyseal part 2. The stem ends with a frustum-shaped portion 9 for the application of the spherical head for the articulation of the endoprosthesis 1 in the cotyloid element which is anchored in the acetabulum of the pelvic bone.

A screw 10 is driven through the collar 7 and the bush 6; once screwed into a threaded hole 11a of the metaphyseal part 2, the screw allows to lock the collar 7 on the bush 6 and therefore determine the angular orientation of the stem 8 with respect to the central plane P. The collar 7 has an outside diameter by way of which it remains internal to the surface that lies between the axis B and the face F1. In this manner, the metaphyseal part 2 protrudes, below the stem 8, with a step 11 which forms a recess 12 together with the stem 8.

The described endoprosthesis has a set of three slits 13, 14 and 15 such that the slit 13 forms an angle of 135° with the other two slits 14, 15 with respect to the axis A. Moreover, the slit 13 lies on the plane P and is open on the face F1, while the other two slits 14 and 15 delimit a sector 16 which comprises the face F2, which remains intact.

It is evident that the endoprosthesis of the invention perfectly achieves the intended aim and objects. The flattened octagonal cross-section, particularly of the metaphyseal portion, gives the prosthesis 1 the ability to adapt to the shaped of the medullary canal, facilitating intimate bonding with the femoral bone. At the same time, the edges of the octagon, while ensuring a valid rotation-preventing effect, do not produce sharp edges which are capable of biting into the bone tissue, triggering degenerative processes affecting the stability of the bone implant.

A particular advantage of the present invention is the axial offset between the axis A of the stem and the axis B of the collar, which reduces the load on the cortices.

A further advantage which also facilitates better distribution of the load on the cortices is offered by the flat face F1, which has a large area thanks to the octagonal shape of the transverse cross-section. The octagonal transverse configuration allows to take advantage of a better stress on the cortices, achieved by axial offset, while maintaining the correct balance between stress on the medial and lateral cortex and continuity of contact with the anterior and posterior cortices so as to render the rod stable with respect to rotational stresses on a transverse plane. The combination of orthogonal arrangement and axial offset therefore allows to achieve a better distribution of loads on the cortices while maintaining an equal rotational stability.

It should be noted that the inner face F1 and the outer face F2, particularly of the sector 16, provide a larger surface for resting on the cortices and therefore generate less pressure. The recess 12 allows, during walking, a distribution of loads and a consequent variation of the moments generated by the muscular stabilization component.

The described endoprosthesis is susceptible of numerous modifications and variations, all of which are within the scope of the same inventive concept.

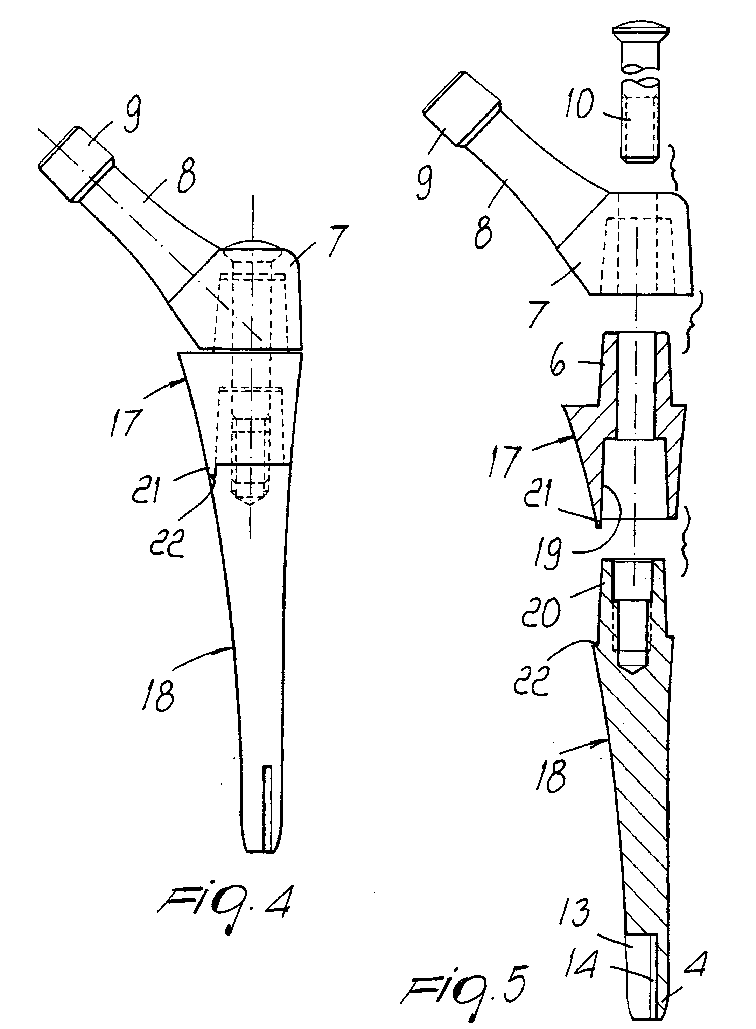

Figures 4 and 5 are views of an embodiment in which the metaphyseal part and the rod constitute two separate modules, a metaphyseal one 17 and a distal one 18, having respectively a conical cavity 19 and a tubular tang 20 which are complementary one another in order to allow coupling by means of the screw 10 which, after passing through the collar 7 and the metaphyseal module 17, engages by screw coupling the threaded hole of the distal module 18. In order to provide further assurance against the rotation of one module 17 with respect to the other module 18, there is a tooth 21 which engages, from the lower edge of the metaphyseal module 17, a notch 22 of the distal module 18, at the base of the tubular tang 20.

By appropriately varying the shape and dimensions of the modules 17 and 18 it is possible to achieve a large number of combinations capable of adapting to the different femoral anatomies.

In the practical execution of the invention, the materials used may be any according to the requirements. Advantageously, the endoprosthesis is made of biocompatible titanium alloy.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. A hip endoprosthesis, comprising a collar (7) and a metaphyseal portion (2) which is blended, on a distal side, with a rod-shaped portion (3) and has, on a proximal side, a taper-fit coupling (5, 6) for the collar (7) of the endoprosthesis from which a coupling stem (8) protrudes obliquely for a spherical head for the articulation of the endoprosthesis to the pelvic bone, **characterized in that** said metaphyseal and rod-shaped portions (2, 3) have an octagonal cross-section which is flattened on the central plane, with two faces which are perpendicular to the main axis of said cross-section, and **in that** said metaphyseal portion (2), below said stem (8), protrudes beyond a peripheral region of said collar (7), forming a step (11) which forms a recess (12) with said stem.

2. The hip endoprosthesis according to claim 1, **characterized in that** the axis (A) of the rod-shaped portion (3) is axially offset from the axis (B) of the taper-fit coupling (5,6).

3. The hip endoprosthesis according to claim 2, **characterized in that** said rod-like portion (3) has an end which is provided with a set of three slits (13-15) forming a sector in which one face is arranged at right angles to the main axis of said cross-section.

4. The hip endoprosthesis according to claim 1, **characterized in that** said metaphyseal portion (2) and said rod-shaped portion (3) constitute two separate metaphyseal and distal modules (17, 18) which respectively have a conical cavity (19) and a tubular tang (20) which are complementary one another in order to allow coupling by means of a screw (10) which is driven through said collar and said metaphyseal module and engages, by screw coupling, in a threaded hole of said distal module.

5. The hip endoprosthesis according to claim 4, **characterized in that** in order to provide better assurance against the rotation of one module with respect to the other, a tooth (21) is provided protruding from a lower edge of said metaphyseal module (17) and engages a notch (22) of the distal module (18).

## Patentansprüche

1. Hüftendoprothese umfassend einen Bund (7) und einen metaphysialen Teil (2), der auf einer distalen Seite mit einem stabförmigen Teil (3) verbunden ist, und auf einer proximalen Seite eine Konuspasskupplung (5, 6) für den Bund (7) der Endoprothese aufweist, von der ein Kupplungsschaft (8) für einen kugelförmigen Kopf für die Gelenkverbindung der Endoprothese mit dem Beckenknochen schräg hervorsteht, **dadurch gekennzeichnet, dass** die metaphysialen und stabförmigen Teile (2, 3) einen octagonalen Querschnitt aufweisen, der auf einer zentralen Ebene abgeflacht ist, mit zwei Seiten, die zur Hauptachse des Querschnitts senkrecht sind, und dadurch, dass der metaphysiale Teil (2) unter dem Schaft (8) über einen peripheren Bereich des Bundes (7) hervorsteht, so dass eine Stufe (11) ausgebildet ist, die eine Ausnehmung (12) in dem Schaft bildet.

2. Hüftendoprothese nach Anspruch 1, **dadurch gekennzeichnet, dass** die Achse (A) des stabförmigen Teils (3) axial von der Achse (B) der Konuspasskupplung (5, 6) versetzt ist.

3. Hüftendoprothese nach Anspruch 2, **dadurch gekennzeichnet, dass** der stabförmige Teil (3) ein Ende aufweist, das mit einem Satz von drei Schlitzen (13-15) versehen ist, die einen Sektor bilden, in dem eine Seite im rechten Winkel zur Hauptachse des Querschnitts angeordnet ist.

4. Hüftendoprothese nach Anspruch 1, **dadurch gekennzeichnet, dass** der metaphysiale Teil (2) und der stabförmige Teil (3) zwei separate metaphysiale und distale Module (17, 18) bilden, die jeweils einen konischen Hohlraum (19) und einen rohrförmigen Teil (20) aufweisen, die komplementär zueinander sind, um eine Kopplung mittels einer Schraube (10) zu ermöglichen, die durch den Bund und den metaphysialen Modul getrieben wird und durch Schraubverbindung in eine Gewindebohrung des distalen Moduls eingreift.

5. Hüftendoprothese nach Anspruch 4, **dadurch gekennzeichnet, dass** um eine bessere Sicherheit gegen Rotation eines Moduls gegen den anderen zu erreichen, ein Zahn (21) vorgesehen ist, der von einer unteren Kante des metaphysialen Moduls (17) hervorsteht und in eine Kerbe (22) des distalen Moduls (18) eingreift.

## Revendications

1. Endoprothèse de la hanche, comprenant un collet (7) et une partie métaphyséale (2) qui est raccordée, du côté distal, à une partie en forme de queue (3) et présente, du côté proximal, un assemblage à ajustement conique (5, 6) pour le collet (7) de l'endoprothèse à partir duquel fait saillie obliquement une tige d'assemblage (8) pour une tête sphérique pour l'articulation de l'endoprothèse à l'os du bassin,
**caractérisée en ce que** lesdites parties métaphyséale et en forme de queue (2, 3) ont une coupe transversale octogonale qui est aplatie sur le plan central, avec deux faces qui sont perpendiculaires à l'axe principal de ladite coupe transversale, et **en ce que** ladite partie métaphyséale (2), en dessous de ladite tige (8), fait saillie au-delà d'une région périphérique dudit collet (7), formant une marche (11) qui définit un évidement (12) avec ladite tige.

2. Endoprothèse de la hanche selon la revendication 1,
**caractérisée en ce que** l'axe (A) de la partie en forme de queue (3) est décalé axialement de l'axe (B) de l'assemblage à ajustement conique (5, 6).

3. Endoprothèse de la hanche selon la revendication 2,
**caractérisée en ce que** ladite partie en forme de queue (3) a une extrémité qui est munie d'un ensemble de trois fentes (13-15) formant un secteur dans lequel une face est agencée à angle droit par rapport à l'axe principal de ladite coupe transversale.

4. Endoprothèse de la hanche selon la revendication 1,
**caractérisée en ce que** ladite partie métaphyséale (2) et ladite partie en forme de queue (3) constituent deux modules métaphyséal et distal séparés (17, 18) qui ont respectivement une cavité conique (19) et un embout tubulaire (20) qui sont complémentaires l'un de l'autre de façon à permettre l'assemblage par une vis (10) qui traverse ledit collet et ledit module métaphyséal et s'engage, par vissage, dans un trou taraudé dudit module distal.

5. Endoprothèse de la hanche selon la revendication 4,
**caractérisée en ce que**, pour fournir une meilleure assurance contre la rotation d'un module par rapport à l'autre, une dent (21) est prévue, faisant saillie d'un bord inférieur dudit module métaphyséal (17) et engage une encoche (22) du module distal (18).
